# EUROPÄISCHE PATENTANMELDUNG

(11) **EP 2 042 215 A1**
(43) Veröffentlichungstag der Anmeldung: **01.04.2009**
(21) Anmeldenummer: 08105439.7
(22) Anmeldetag: 25.09.2008
(51) Int. Cl.: A61N 5/06, A61F 7/00

(54) **Heizeinrichtung zur Wärmebehandlung eines Menschen**

(30) Priorität: 27.09.2007 AT 5802007 U
(71) Anmelder: Sperrer, Ernst, 2640 Gloggnitz (AT)
(72) Erfinder: Sperrer, Ernst, 2640 Gloggnitz (AT)
(74) Vertreter: Müllner, Martin

(57) **Zusammenfassung**

Heizeinrichtung zur Wärmebehandlung eines Menschen, insbesondere zum Einbau in eine Infrarotkabine, welche Zonen mit unterschiedlicher Wärmestrahlung aufweist. Um einen einfachen Aufbau einer solchen Heizeinrichtung (3) zu ermöglichen, ist vorgesehen, dass die Heizeinrichtung als Flächenstrahler ausgebildet ist, der Zonen (4, 5, 6) mit erhöhter Wärmestrahlung aufweist, wobei die Zonen (4, 5, 6) mit erhöhter Wärmestrahlung zur Bestrahlung der Wirbelsäule, des Hüftbereichs und des Schulterbereichs insgesamt im Wesentlichen I-förmig ausgebildet sind. Vorzugsweise ist ein Sensor zur Erfassung der Größe eines zu bestrahlenden Menschen vorgesehen, der zur Steuerung der Zonen (4, 5, 6) der Heizeinrichtung (3) vorgesehen sind. Bei einer Infrarotkabine mit einer Sitzeinrichtung, die eine Rückenlehne aufweist, und mit solch einer Heizeinrichtung (3) soll diese Heizeinrichtung (3) hinter der Rückenlehne etwa parallel zu dieser eingebaut sein.

## Beschreibung

### Technisches Gebiet

Die Erfindung bezieht sich auf eine Heizeinrichtung gemäß dem Oberbegriff des Anspruchs 1 sowie auf eine Infrarotkabine mit solch einer Heizeinrichtung.

### Stand der Technik

Eine Heizeinrichtung der eingangs erwähnten Art wurde z.B. durch die AT 501123 B bekannt. Bei dieser bekannten Heizeinrichtung sind zwei verschiedene Heizelementtypen vorgesehen, wobei diese Heizelementtypen unterschiedliche Temperaturen aufweisen. Mit dieser bekannten Heizeinrichtung ist es möglich, bestimmte Körperpartien (nämlich die Wirbelsäule und den dieser benachbarten Bereich) einer gezielten Wärmebehandlung zuzuführen und mit dem zweiten Heizelementtyp für eine ausreichende Umgebungstemperatur zu sorgen. Dabei handelt es sich einerseits um einen Flächenstrahler, der für die entsprechende Umgebungstemperatur sorgt, und um Röhrenheizkörper, mit dem die bestimmten Körperpartien einer Wärmebehandlung unterzogen werden können.

Bei dieser bekannten Lösung ergibt sich jedoch der Nachteil, dass die einzelnen Bereiche der Heizeinrichtung bereits bei der Herstellung festgelegt sind und im Wesentlichen aus einem oder mehreren senkrechten Streifen bestehen (wegen der Röhrenheizkörper). Im bekannten Fall sind zwei vertikale verlaufende Streifen mit erhöhter Wärmestrahlung vorgesehen (für die Wirbelsäulen von zwei Personen, die nebeneinander sitzen).

Im Rahmen der vorliegenden Erfindung wurde gefunden, dass sich damit jedoch nicht vermeiden lässt, dass einerseits auch innere Organe (die Nieren) einer unerwünschten Wärmebelastung ausgesetzt werden, aber andererseits andere Körperbereiche, für die eine erhöhte Wärmebestrahlung günstig wäre, nicht ausreichend bestrahlt werden.

Problematisch bei Röhrenstrahlern ist nämlich, dass diese auf sehr geringer Fläche (genau hinter der Wirbelsäule) eine hohe Strahlung entwickeln, um überhaupt die Schulter zu erreichen oder um in dieser kleinen Körperzone (Wirbelsäule) genügend Temperatur für eine ausreichende Tiefenwirkung zu erlangen, damit sich der ganze Körper erwärmt. Temperaturen von 300° sind bei Röhrenstrahlern daher keine Seltenheit, aber diese belasten Haut und Körper.

### Darstellung der Erfindung

Ziel der Erfindung ist es, eine Heizeinrichtung der eingangs erwähnten Art vorzuschlagen, die diese Nachteile vermeidet und sich durch einen einfachen Aufbau auszeichnet.

Erfindungsgemäß wird dies durch die kennzeichnenden Merkmale des Anspruchs 1 erreicht.

Durch die vorgeschlagenen Maßnahmen ist sichergestellt, dass einerseits in verschiedenen Bereichen eine Oberflächentemperatur erreicht wird, die eine Tiefenerwärmung ermöglicht, und andererseits eine unerwünscht hohe Erwärmung der Umgebung vermieden wird. Es werden zusätzlich zur Wirbelsäule insbesondere der Schulter- und der Hüftbereich stärker bestrahlt, der Nierenbereich ist jedoch davon ausgenommen, er wird nur gering bestrahlt (d.h. er liegt in einer Zone mit nicht erhöhter Wärmestrahlung). Im Gegensatz zur AT 501123 B sind nicht zwei Heizelementtypen notwendig, sodass der Aufbau vereinfacht ist und die Nachteile der Röhrenstrahler entfallen.

Um eine leichte Anpassung an die jeweiligen Erfordernisse zu ermöglichen, ist es vorteilhaft, die Merkmale des Anspruchs 2 vorzusehen.

Um eine Anpassung der Zonen der Heizeinrichtung an die Größe des zu behandelnden Menschen zu ermöglichen, können die Merkmale des Anspruchs 3 vorgesehen werden.

Bei einer Infrarotkabine mit einer Sitzeinrichtung, die eine Rückenlehne aufweist, und mit einer erfindungsgemäßen Heizeinrichtung, soll diese hinter der Rückenlehne etwa parallel zu dieser eingebaut sein. Auf diese Weise wird einerseits ein bequemes Sitzen (wegen der Rückenlehne) ermöglicht und andererseits dennoch eine gleichmäßige Bestrahlung über die gesamte Höhe des Rückens erzielt (gleichmäßig natürlich nur abgesehen von den Zonen mit erhöhter Wärmestrahlung), weil der Abstand zwischen dem Rücken und dem Wärmestrahler etwa konstant ist.

### Kurze Beschreibung der Abbildungen der Zeichnungen

Die Erfindung wird nun anhand der Zeichnung näher erläutert. Dabei zeigt:

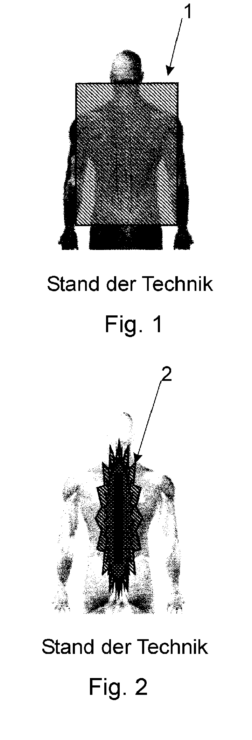

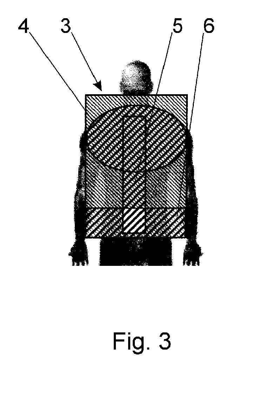

Fig. 1 schematisch einen herkömmlichen Flächenstrahler; Fig. 2 schematisch einen herkömmlichen Röhrenstrahler; und Fig. 3 schematisch eine erfindungsgemäße Heizeinrichtung.

### Bester Weg zur Ausführung der Erfindung

Für eine wirkungsvolle Anwendung von Infrarotstrahlungswärme ist eine kontinuierliche Infrarotwärmestrahlenzufuhr die erste Voraussetzung. Diese kann über einen guten Speicherkern der Heizeinrichtung und einer Regelung der Infrarotwärmestrahler über eine Steuerung erfolgen. Dadurch ist eine ständige Zufuhr von Infrarotwärmestrahlen gewährleistet, wobei gleichzeitig die Intensität auf momentane Bedürfnisse des Anwenders eingestellt werden kann. Die Temperatur des Infrarotwärmestrahlers bestimmt einerseits die Infrarotwellenlänge und damit die Eindringtiefe in die Haut und andererseits die Körper- und Hautbelastung. Um eine geringe Haut- und Körperbelastung zu erwirken, ist der Infrarot-C-Bereich der wichtigste Bereich. Um eine entsprechende Infrarotwärmewirkung in diesem Bereich zu erhalten, ist eine kontinuierliche Infrarotstrahlenzufuhr über Speicherkern erforderlich, die durch eine entsprechende Steuerung der Heizleistung sichergestellt werden kann.

Bei der Heizeinrichtung 1 nach Fig. 1 handelt es sich um einen normalen Flächenstrahler. Dieser strahlt die Infrarotstrahlungswärme über eine große Fläche ab und weist eine zum Strahlen geeignete Oberfläche auf. Solche Heizeinrichtungen werden in der Regel mit geringer Temperatur von z.B. 60 bis 80°C betrieben, wobei jedoch nur eine geringe Tiefenwärmewirkung, wenn überhaupt auftritt. Solche Flächenstrahler weisen gelegentlich einen Überzug aus Kunststoff auf.

Bei der Heizeinrichtung 2 nach Fig. 2 handelt es sich um einen Röhrenstrahler. Ein solcher zeichnet sich durch hohe Oberflächentemperaturen aus, wodurch es zu einer entsprechenden Infrarotwärmestrahlung kommt. Das führt zu einer hohen Haut- und Körperbelastung im Bereich der Wirbelsäule und im Nierenbereich. Außerdem kann ein solcher Strahler nur für sehr begrenzte Bereiche eingesetzt werden, wenn er in einer Infrarotkabine eingesetzt wird, da sonst die Temperatur in der Kabine zu hoch werden würde.

In Fig. 3 ist nun eine erfindungsgemäße Heizeinrichtung 3 dargestellt. Diese ist ebenfalls ein Flächenstrahler. Allerdings weist dieser Zonen 4, 5 und 6 auf, in denen höhere Oberflächentemperaturen als in den übrigen Bereichen des Strahlers einstellbar sind. Diese Zonen 4, 5, 6 sind insgesamt I-förmig angeordnet. Diese Zonen 4, 5, 6 können z.B. dadurch ausgebildet werden, dass vor einem Speicherkern des Flächenstrahlers individuell ansteuerbare Heizelemente angeordnet sind. Dadurch wird in den angegebenen Bereichen eine höhere Oberflächentemperatur erreicht, die zu einer entsprechend erhöhten Infrarotwärmestrahlung in den Bereichen 4, 5, 6 führt.

Dadurch ist es möglich, z.B. im Schulter- und Gesäßbereich wie auch im Bereich der Wirbelsäule eine entsprechende Infrarotwärmestrahlung anzuwenden. Da diese Bereiche entsprechend begrenzt sind, kommt es zu keiner zu starken Erhöhung der Temperatur der Umgebung, was insbesondere bei einem Einbau einer solchen Heizeinrichtung 3 in eine Infrarotkabine von Bedeutung ist.

Diese Bereiche 4, 5 und 6 sind zweckmäßigerweise mittels einer nicht dargestellten PI-I Steuerung individuell steuerbar.

Es ist günstig, die Größe der Person, die sich bestrahlen lässt, mit einem Sensor (nicht dargestellt) zu erfassen. Der Bereich zur Erwärmung des Schultergürtels kann dann für kleine Personen etwas nach unten versetzt werden, was wiederum dadurch erreicht werden kann, dass andere Bereiche des Flächenstrahlers stärker aufgeheizt werden. Es wird also nichts mechanisch verschoben, es werden nur - je nach Größe der Person - andere Bereiche stärker beheizt.

## Patentansprüche

1. Heizeinrichtung zur Wärmebehandlung eines Menschen, insbesondere zum Einbau in eine Infrarotkabine, welche Zonen mit unterschiedlicher Wärmestrahlung aufweist, **dadurch gekennzeichnet, dass** die Heizeinrichtung (3) als Flächenstrahler ausgebildet ist, der Zonen (4, 5, 6) mit erhöhter Wärmestrahlung aufweist, wobei die Zonen (4, 5, 6) mit erhöhter Wärmestrahlung zur Bestrahlung der Wirbelsäule, des Hüftbereichs und des Schulterbereichs insgesamt im Wesentlichen I-förmig ausgebildet sind.

2. Heizeinrichtung nach Anspruch 1, **dadurch gekennzeichnet, dass** die Temperatur in den verschiedenen Zonen (4, 5, 6) individuell steuerbar ist.

3. Heizeinrichtung nach einem der Ansprüche 1 oder 2, **dadurch gekennzeichnet, dass** ein Sensor zur Erfassung der Größe eines zu bestrahlenden Menschen vorgesehen ist, der zur Steuerung der Zonen (4, 5, 6) der Heizeinrichtung (3) vorgesehen ist.

4. Infrarotkabine mit einer Sitzeinrichtung, die eine Rückenlehne aufweist, und mit einer Heizeinrichtung, **dadurch gekennzeichnet, dass** die Heizeinrichtung gemäß den Ansprüchen 1-3 ausgebildet ist und hinter der Rückenlehne etwa parallel zu dieser eingebaut ist.
